# EUROPEAN PATENT SPECIFICATION

(11) **EP 2 090 280 B1**
(45) Date of publication and mention of the grant of the patent: **16.10.2019**
(21) Application number: 08101707.1
(22) Date of filing: 18.02.2008
(51) Int. Cl.: A61K 6/02

(54) **Two-component composition for filling an implant abutment**
Zwei-Komponenten-Zusammensetzung zur Füllung einer Implantatstütze
Composition à deux composants pour le remplissage d'une butée d'implant

(43) Date of publication of application: 19.08.2009
(73) Proprietor: Coltène/Whaledent AG, 9450 Altstaetten (CH)
(72) Inventor: Kollefrath, Ralf, 9464 Ruethi (CH); Mannschedel, Werner, 89129 Langenau (DE)
(74) Representative: Hepp Wenger Ryffel AG

(56) References cited:
- EP-A- 1 787 627
- WO-A-2006/045646
- WO-A1-98/16168
- US-A- 6 120 294
- US-A1- 2004 044 164
- US-B1- 6 852 822
- "Annexe 1", , 17 January 2006 (2006-01-17), Retrieved from the Internet: URL:http://www.osseonews.com/filling-the-s crew-channel/print/ [retrieved on 2012-04-03]
- "Annexe 2", , Retrieved from the Internet: URL:http://products.dentalproductsreport.c om/community/DisplayAd.asp?id=3842 [retrieved on 2012-04-03]

## Description

The invention relates to a two-component composition as defined in the claims for use in a prophylaxis and/or treatment of bacterial infiltrations comprising the step of filling an implant abutment.

Implants for the replacement of roots of teeth have been long known in dentistry. Such an implant comprises an implant post, which is fixed to a jaw bone, and an implant abutment, which is attached to the implant post, for example with the help of a screw. This screw is inserted through an opening in the implant abutment and screwed into the implant post. This opening, in particular the screw hole, is usually at least partially filled with a filling material, such as provisional cement, a cotton pellet, or so-called guttapercha. Alternatively, compositions based on acrylates and curing in situ have been used for this purpose. A denture, such as a prosthesis, crown, or bridge, is finally fixed to the filled implant abutment with the help of a dental cement.

However, the above mentioned materials have some major drawbacks: For example, acrylates typically undergo a shrinkage of at least several percent during curing, which leaves open cavities in the abutment opening, thereby allowing the inclusion of bacteria, which might lead to infections. Infections may also occur when cotton pellets are used, which may contain bacteria in. their porous structure. Moreover, acrylates currently used require curing by radiation, for example UV radiation, which necessitates further apparatus.

Furthermore, in certain cases, the denture and the filling material may have to be removed again, for example when the implant abutment needs to be cleaned or replaced or when the screw fixing the implant abutment to the implant post needs to be replaced or retightened, or when the implant post has to be replaced. Cured acrylates and provisional cements which are used as a filling material have a tendency to stick to all common composite or resin reinforced glass ionomer dental cements based on acrylates. Additionally, commonly used acrylates tend to stick also to the implant abutment. This causes the filling material to tear apart when it is to be removed, thereby complicating the removal of the filling material. Moreover, guttapercha has to be applied warm or hot in order to be deformable.

The bacterial infiltration issues linked to the presence of dental implants is already known in the art. In the common practice, the skilled person uses available one component dental filling materials.

Document WO 98/16168 discloses dental implants and dental systems having a dental abutment. The open spaces of the abutments are sealed in order to prevent the bacterial colonisation. The sealing can either be obtained by placing a plug in the opening or injecting a paste like curable composition in the opening. There is no indication about a two-component composition.

Each of Documents WO 2006/045646, US A 6,120,294, EP 1 787 628, US B1 6 852 822 and US 2004/044164A1 discloses two-component dental filling silicone compositions but none of these documents discloses the step of filling a dental abutment.

It is therefore an object of the present invention to avoid the disadvantages of the prior art, especially to provide a composition for use in a prophylaxis and/or treatment of bacterial infiltrations comprising the step of filling an implant abutment which is easy to apply, does not stick to common dental cements, and which does not leave substantial open cavities in the abutment opening.

This object is solved by a two-component composition for use in a prophylaxis and/or treatment of bacterial infiltrations comprising the step of filling an opening in an implant abutment according to the independent claim. The composition may be used for substantially, in particular completely filling a central axial opening in an implant abutment, most preferably a screw hole in an implant abutment. The composition is a two-component silicone composition.

Within the context of this application, a two-component composition is understood as a composition comprising two separately stored components, which undergo a curing reaction, in particular a crosslinking reaction, when they are brought into contact with each other at the time of the intended use of the composition, without the need for any external influences required for initiating the curing, such as radiation or heat.

The use of such a two-component material allows an optimal filling of an opening in an implant abutment, such as a screw hole in an implant abutment. Therefore, there is no danger of an inclusion of bacteria or other contaminants which could cause infections or other adverse effects to a patient's health. Thus, the use of the two-component material according to the invention is particularly suitable for and therefore used for the prophylaxis and/or treatment of bacterial infiltrations. In addition, the use of a two-component material allowed to cure in situ makes further apparatus for initiating the curing reaction, such as light sources, redundant. Furthermore, even after curing, the silicone material does not bond to common composite or resin reinforced glass ionomer dental cements such as Ketac Cem (available from 3M ESPE), FujiCEM (available from GC), or ParaCem (available from Coltène/Whaledent). Therefore, a denture fixed to the implant abutment and the filling material contained therein by a common dental cement can be removed again from the cured composition without difficulty - contrary to the known composition based on acrylates. Preferably, the cured two-component composition is essentially not flowable so that it does not migrate.

Within the scope of the invention, the opening in the implant abutment does not necessarily have to be a screw hole. For example, in an alternative embodiment, the implant abutment may have a thread itself, by which it can be fixed to the implant post. For this purpose, the implant abutment has an opening for inserting a tool, such as a screw driver, for screwing the implant abutment into or onto the implant post. This opening for the tool may also be substantially, in particular completely filled with the composition of the invention.

According to the invention, the silicone composition is crosslinkable by an addition reaction. The use of silicone materials crosslinkable by addition reactions in dentistry is known per se and avoids possible adverse effects to the health of the patients since no harmful compounds are released during curing. Suitable silicone compositions crosslinkable by an addition reaction are commercially available in vast variety in the dental industry. With respect to the chemistry of suitable silicones, it is referred to "Chemie und Technologie der Silicone" (Walter Noll, Verlag Chemie, 1960).

According to the invention, the composition exhibits only a slight change of volume upon curing: In one embodiment, it may exhibit a volume reduction of less than 1 %, preferably less than 0.5 %, most preferably less than 0.2 % upon curing. The use of a composition exhibiting a volume reduction as above has the effect that essentially the entire opening is filled and remains entirely filled during curing. Hence, no substantial cavities are formed, even when it has cured, unlike it is the case when a conventional acrylate is used. Therefore, the danger of an inclusion of bacteria or other contaminants is even more decreased.

In another embodiment, the composition may exhibit a volume expansion, preferably below 1 %, more preferably below 0.5 %, most preferably below 0.2 %. Such a volume expansion can lead to an enhanced tightness of fit, in particular at the marginal seal. Such compositions are known per se and readily available to the person having ordinary skill in the art, e. g. based on DE 3929081 A1, DE 2906214 A1, or US 4871782.

Preferably, the curing reaction is terminated after 24 hours, preferably after 3 hours, most preferably after 15 minutes. When the curing reaction has terminated, the composition remains essentially dimensionally stable.

In a preferred embodiment, the composition has a Shore hardness in the cured state of between 10 Shore A and 80 Shore D, preferably between 25 Shore A and 70 Shore D, most preferably between 40 Shore A and 60 Shore D. Compositions possessing this property are stable enough in order to be replaced in one piece; at the same time, they are soft enough in order to absorb to a sufficient extent any outer pressure exerted onto the denture and thereby onto the filling material.

The composition may contain further additives. In particular, it may contain at least one bacteriostatic agent. Suitable bacteriostatic agents are, for example, silver, zinc oxide, copper, quarternary ammonia salts, fluorides, or any of those disclosed in EP 1 652 507, EP 1 787 627, US 2004/0091558, WO 2005/058238, or US 2006/0159630 or combinations thereof. These bacteriostatic agents serve to further prevent bacterial infiltrations.

The composition may further contain one or more of the following additives: rheologically active additives, additives for improving the surface wettability such as tensides, additives for improving the surface adhesion such as silanes, or pigments for altering the visual appearance.

A further aspect (which is disclosed, but not claimed as such) is a method of substantially, in particular completely filling an opening in an implant abutment with a two-component composition, in particular with a composition according to the present invention. The method includes the steps of
a) applying the components of the composition to the opening in the implant abutment;
b) allowing the composition to cure in situ.

Here and hereafter, "in situ" refers to the position in an opening in an implant abutment, in particular in a patient's mouth. Preferably, within the above method, a central axial opening in an implant abutment, most preferably a screw hole in an implant abutment is substantially, in particular completely filled.

The use of a two-component material allows an optimal filling of an opening in an implant abutment, such as a screw hole in an implant abutment. Furthermore, the use of a two-component material allowed to cure in situ makes further apparatus for initiating the curing reaction, such as light sources, redundant. Moreover, especially silicone compositions are superior to acrylate compositions since they do not stick to common dental cements used for fixing a denture, which significantly facilitates the removal: The cured composition of the current invention does not tear apart and allows removal of the filling material in one piece.

Advantageously, the components for the production of the composition are provided in a multi-chamber cartridge system, preferably in a two-chamber cartridge system. The application to the implant abutment may be performed via a mixing device, preferably by a static mixing device, which may be comprised in the multi-chamber cartridge system. Multi-chamber cartridge systems and static mixing devices are well known to a person having ordinary skill in the art. The static mixing device may comprise a mixing tip having an implemented and/or attached and/or attachable nozzle suitable to apply the mixed composition directly into an opening in a common implant abutment to be filled. The nozzle may comprise a metal and/or plastics such as polypropylene. Advantageously, the nozzle is elastic in order to facilitate the application of the composition. Preferably, the nozzle is dimensioned to fit into an opening in a common implant abutment. In particular, the nozzle may contain a tubular end portion having a diameter of 1 mm or less.

The invention is explained in more detail by a non-limiting example and figures, wherein
- Figure 1: shows a sectional view of an implant abutment with unfilled screw hole;
- Figure 2: shows a sectional view of the implant abutment of Figure 1 with filled screw hole;
- Figure 3: shows a sectional view of the implant abutment of Figures 1 and 2 with filled screw hole and an applied denture;
- Figure 4: shows a part of a mixing device and an attached nozzle for application of the composition;
- Figure 5: shows the nozzle of Figure 4 in an enlarged view.

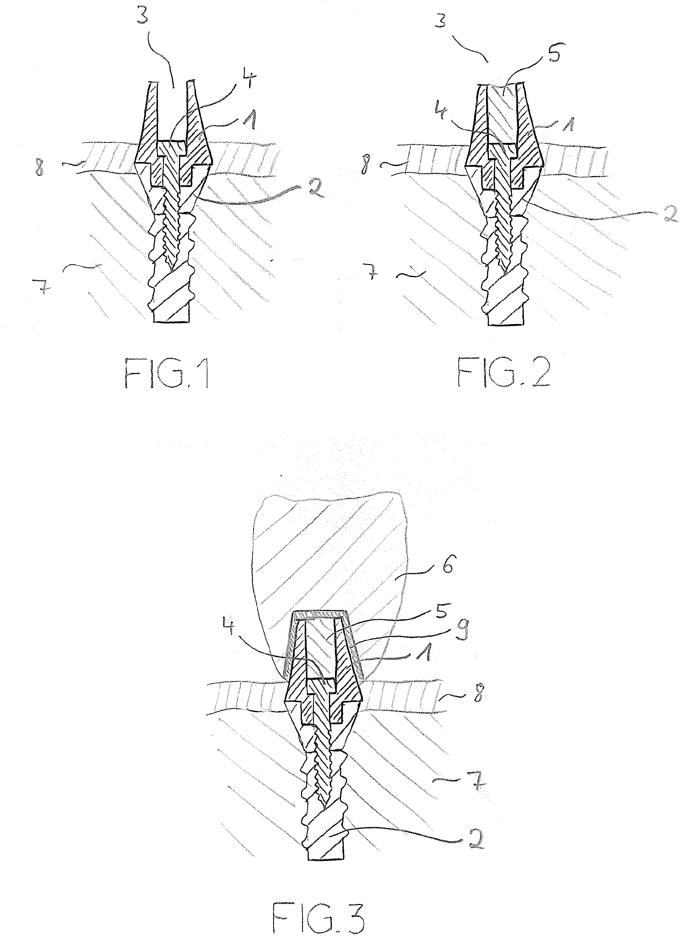

Figure 1 shows an implant post 2 fixed within a jaw bone 7. An implant abutment 1 is fixed on top of the implant post 2 with the help of a screw 4. This screw 4 is inserted into a screw hole 3, which forms an opening in the implant abutment 1. The jaw bone 7 is covered by gingiva 8, which surrounds the implant post 2 and the implant abutment 1.

The screw hole 3 in the implant abutment 1 may be filled with a composition 5 according to the present invention and containing a two-component silicone by applying the components of the composition 5 to the screw hole 3, thereby arriving at the situation depicted in Figure 2. The two components A and B for the production of the composition 5 may be applied from a common two-chamber cartridge system and a static mixing device, which are not shown explicitly. The composition 5 may be allowed to cure in situ, i.e. in the screw hole 3.

Component A may consist of:

| | |
|---|---|
| 10 g | α,ω-divinyl-polydimethylsiloxane (viscosity 20 Pas; Wacker Silicones) |
| 5 g | quartz powder Sikron B600 (Quarzwerke Frechen, Germany) |
| 0.05 g | Silopren U-catalyst Pt/D (GE Bayer Silicones) |
| 0.02 g | divinyltetramethyldisiloxane (Fluka). |

The components are intimately mixed.

Component B may consist of:

| | |
|---|---|
| 9.8 g | α,ω-divinyl-polydimethylsiloxane (viscosity 20 Pas; Wacker Silicones) |
| 5 g | quartz powder Sikron B600 (Quarzwerke Frechen, Germany) |
| 0.3 g | polymethylhydrosiloxane (viscosity 20 mPas; Wacker Silicones). |

The components are likewise intimately mixed.

Both components A and B are dispensed simultaneously and homogeneously mixed by a static mixing device at a mass ratio of 1:1. However, other mass ratios of up to 1:10 or 10:1 are also conceivable. Due to the mixing of the silicone components A and B shortly before the application, the curing reaction proceeds in situ. In particular, no additional apparatus for curing, such as light sources, are required, as it is the case for acrylate compositions.

During curing, the composition exhibits a volume reduction of 0.2 %. Due to this volume reduction, no significant cavities are left open in the screw hole 3 upon curing, as it is the case when a conventional acrylate composition is used.

When the composition 5 is at least partially, preferably fully cured, a denture 6 is fixed to the implant abutment 1 filled with a -composition 5 with the help of a dental cement 9, as shown in Figure 3.

When the denture 6 has to be removed again, as for example when the screw 4 fixing the implant abutment 1 to the implant post 2 needs, to be replaced or retightened or when the implant post has to be replaced, the composition 5 will not stick to the dental cement 9, whereby the cured composition 5 is not torn apart. Instead, the denture 6 can be removed together with only the dental cement 9 sticking to it, but not with the composition 5. In a next step, the cured composition 5 may be removed from the implant abutment 1 in one piece. Finally, the screw 4 may be replaced or retightened and/or the implant post may be replaced.

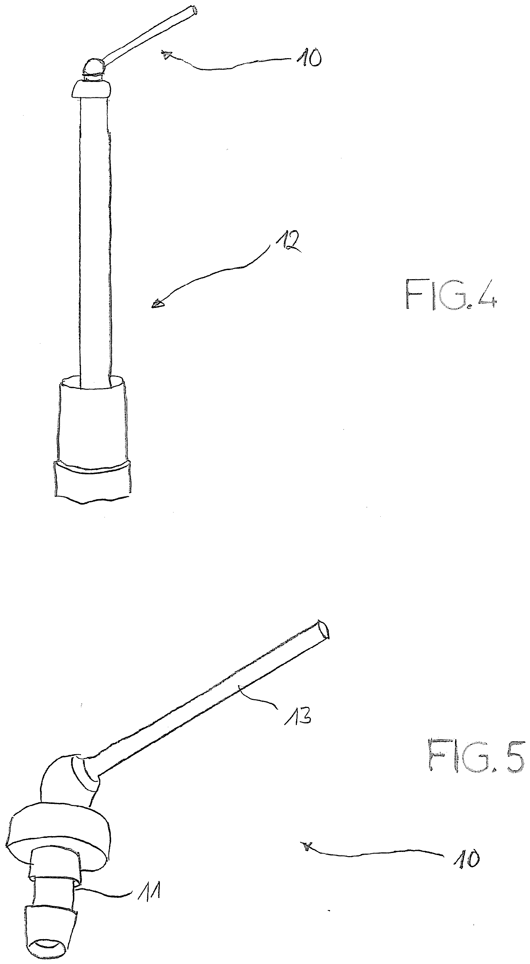

Figure 4 shows a part of a mixing device 12 and an attached nozzle 10 for application of the composition. An enlarged view of the nozzle 10 is shown in Figure 5. It comprises a connecting portion 11 for connecting it to the mixing device 12 and a tubular end portion 13. The end portion 13 is elastic and consists of polypropylene; it has a length of 1.5 mm and a diameter of 1 mm. These properties are convenient for applying the composition to an opening in a common implant abutment.

## Claims

1. Two-component composition (5) for use in a prophylaxis and/or treatment of bacterial infiltrations comprising the step of filling an opening in an implant abutment, wherein the two-component composition (5) exhibits a volume expansion, in particular a volume expansion of below 1 %, or a volume reduction, in particular a volume reduction of less than 1 %, wherein the two-component composition (5) is a silicone composition (5) that is crosslinkable by an addition reaction.

2. Composition (5) according to claim 1,
**characterized in that**
the two-component composition (5) has a Shore hardness in the cured state of between 10 Shore A and 80 Shore D.

3. Composition (5) according to one of the preceding claims,
**characterized in that**
at least one of the components of the two-component composition (5) contains a bacteriostatic agent.

## Patentansprüche

1. Zweikomponentenzusammensetzung (5) zur Verwendung bei der Prophylaxe und/oder Behandlung von Bakterieninfiltrationen, umfassend den Schritt des Füllens einer Öffnung in einem Implantat-Abutment, wobei die Zweikomponentenzusammensetzung (5) eine Volumenexpansion zeigt, insbesondere eine Volumenexpansion von unter 1%, oder eine Volumenreduktion zeigt, insbesondere eine Volumenreduktion von weniger als 1%, wobei es sich bei der Zweikomponentenzusammensetzung (5) um eine Silikonzusammensetzung (5) handelt, die durch eine Additionsreaktion quervernetzbar ist.

2. Zusammensetzung (5) nach Anspruch 1,
**dadurch gekennzeichnet, dass**
die Zweikomponentenzusammensetzung (5) im gehärteten Zustand eine Shore-Härte zwischen 10 Shore A und 80 Shore D aufweist.

3. Zusammensetzung (5) nach einem der vorhergehenden Ansprüche, **dadurch gekennzeichnet, dass** wenigstens eine der Komponenten der Zweikomponentenzusammensetzung (5) ein Bakteriostatikum enthält.

## Revendications

1. Composition à deux constituants (5) destinée à une utilisation dans une prophylaxie et/ou un traitement d'infiltrations bactériennes comprenant l'étape de remplissage d'une ouverture dans un tenon d'implant, la composition à deux constituants (5) présentant une augmentation de volume, en particulier une augmentation de volume inférieure à 1 %, ou une réduction de volume, en particulier une réduction de volume inférieure à 1 %, la composition à deux constituants (5) étant une composition de silicone (5) qui est réticulable par une réaction d'addition.

2. Composition (5) selon la revendication 1,
**caractérisée en ce que**
la composition à deux constituants (5) a une dureté Shore à l'état durci de 10 Shore A à 80 Shore D.

3. Composition (5) selon l'une quelconque des revendications précédentes,
**caractérisée en ce que**
au moins un des constituants de la composition à deux constituants (5) contient un agent bactériostatique.
